# DEMANDE DE BREVET EUROPEEN

(11) **EP 0 953 331 A2**
(43) Date de publication de la demande: **03.11.1999**
(21) Numéro de dépôt: 99400891.0
(22) Date de dépôt: 12.04.1999
(51) Int. Cl.: A61K 7/02, A61K 7/48

(54) **Composition anhydre fluorée et produits cosmétiques ou de soins la contenant**

(30) Priorité: 15.04.1998 FR 9804683; 30.06.1998 FR 9808339
(71) Demandeur: L'OREAL, 75008 Paris (FR)
(72) Inventeur: Bara, Isabelle, 75013 Paris (FR)
(74) Mandataire: Lhoste, Catherine

(57) **Abrégé**

Composition anhydre et homogène comprenant en association au moins une huile fluorée et au moins une cire fluorée à squelette carboné.

Cette composition trouve une application dans la préparation de produits de maquillage ou de soins.

## Description

La présente invention a pour objet une composition anhydre et homogène comprenant en association au moins une huile fluorée et au moins une cire fluorée à squelette carboné.

La présente invention a également pour objet des produits de maquillage ou de soins contenant une telle composition anhydre.

L'utilisation d'huiles fluorées dans la formulation de compositions cosmétiques est particulièrement recherchée dans la mesure où les huiles fluorées confèrent de bonnes propriétés filmogènes et de résistance à l'eau et au sébum.

De telles compositions sont toutefois difficilement réalisables du fait de l'incompatibilité inhérente des huiles fluorées avec de nombreux composés hydrocarbonés polaires et apolaires.

Des associations homogènes d'huiles fluorées et de cires ont déjà été réalisées à l'aide de tensioactifs, mais jusqu'à présent il n'a pas été possible d'obtenir des compositions notamment anhydres à base d'huiles fluorées en l'absence de tensioactifs.

Après de nombreuses études sur divers types de composés, on a constaté de façon surprenante et inattendue qu'en associant certaines huiles fluorées à au moins une cire fluorée à squelette carboné, on pouvait réaliser des mélanges anhydres d'une parfaite homogénéité.

On a par ailleurs constaté que cette association particulière pouvait avantageusement constituer la phase grasse de diverses compositions de maquillage et de soins présentant notamment une excellente texture et conférant à la peau, après application, une grande douceur et un toucher agréable.

La présente invention a donc pour objet une composition ou phase anhydre et homogène comprenant en association au moins une huile fluorée et au moins une cire fluorée à squelette carboné, éventuellement interrompu par un atome d'oxygène.

On entend par l'expression "cire fluorée à squelette carboné" un composé solide à température ambiante ayant un point de fusion supérieur ou égal à environ 30°C et comportant un nombre d'atomes de fluor de préférence supérieur à celui des atomes d'hydrogène, le squelette carboné pouvant être interrompu par au moins un atome d'oxygène. Cette cire ne comporte que des atomes de fluor, d'hydrogène et de carbone et éventuellement d'oxygène.

Par huile, on entend un composé non aqueux liquide, à température ambiante (25°C).

Parmi les huiles fluorées compatibles avec les cires fluorées à squelette carboné, on peut citer notamment les composés perfluorés, les silicones fluorées, les composés fluoroalkyles et hétérofluoroalkyles.

Par composés perfluorés, on entend selon l'invention, des composés dont tous les atomes d'hydrogène ont été substitués par des atomes de fluor.
**A** - Parmi les composés perfluorés répondant à cette définition, on peut notamment citer :
   1) les perfluoropolyéthers répondant aux formules (l) et (ll) suivan-tes; 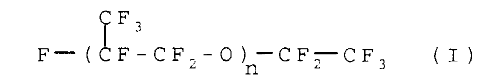 dans laquelle :
      n est 7 à 30 ; et 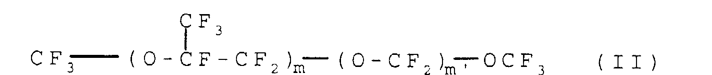
      le rapport m/m' étant de 20 à 40, et
      le poids moléculaire étant de 500 à 20000.

      Parmi ces perfluoropolyéthers de formules (l) et (ll), on peut respectivement citer celui vendu sous la dénomination de "FLUORTRESS LM36^{®}" par la Société DUPONT, et ceux vendus sous la dénomination générale de "FOMBLIN" par la Société MONTEFLUOS par exemple FOMBLIN HC R^{®} de tension vapeur 10⁻⁷ mm Hg.
   2) les composés perfluorocycloalkyles de formule (III) suivante :

      (CF₂)ₑ (CF-CCF₂)ₚ-F)_{g} (III)

      dans laquelle :
      e est égal à 4 ou 5,
      g est égal à 1 ou 2, et
      p est égal à 1, 2 ou 3 ;
      sous réserve que lorsque g=2, les groupements ne sont pas nécessairement en alpha l'un par rapport à l'autre.
      Parmi les composés de formule (III), on peut notamment citer le perfluorométhylcyclopentane et le perfluorodiméthylcyclohexane, vendus respectivement sous les dénominations de "FLUTEC PC1^{®}" de pression de vapeur de 368 mbar et "FLUTEC PC3^{®}" par la Société BNFL FLUOROCHEMICALS Ltd. ; et
   3) le perfluorophénanthrène vendu sous la dénomination de "FLUTEC PC11^{®}" par la Société BNFL FLUOROCHEMICALS Ltd.

**B** - Parmi les silicones fluorées, on peut notamment citer les composés fluoro-siliconés répondant à la formule (IV) suivante: 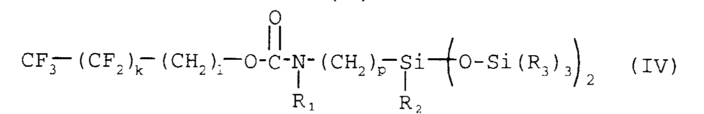 dans laquelle :
   k est 1 à 17,
   l est 1 à 18,
   p est 1 à 6, et
   R₁ représente un atome d'hydrogène ou un radical alkyle en C₁-C₆,
   R₂ représente un radical alkyle en C₁-C₆ ou le radical -OSi(R₃)₃, et
   R₃ représente un radical alkyle en C₁-C₄.

   Parmi les composés répondant à la formule (IV), on peut notamment citer :
   - le N-(-2-F-octyl-éthyloxycarbonyl)-3-aminopropyl bis(triméthylsiloxy)méthylsilane,
   - le N-(-2-F-hexyl-éthyloxycarbonyl)-3-aminopropyl bis(triméthylsiloxy)méthylsilane,
   - le N-(-2-F-butyl-éthyloxycarbonyl)-3-aminopropyl bis(triméthylsiloxy)méthylsilane,
   - le N-(-2-F-octyl-éthyloxycarbonyl)-3-aminopropyl tris(triméthylsiloxy)silane,
   - le N-(-2-F-hexyl-éthyloxycarbonyl)-3-aminopropyl tris(triméthylsiloxy)silane, et
   - le N-(-2-F-butyl-éthyloxycarbonyl)-3-aminopropyl tris(triméthylsiloxy)silane.
**C** - Parmi les composés fluoroalkyles ou hétérofluoroalkyles, on peut citer ceux répondant à la formule (V) suivante:

   CH₃-(CH₂)ₕ-[Z]ₜ-(CF₂)ⱼ-CF₃ (V)

   dans laquelle :
   t est égal à 0 ou 1,
   h est égal à 0, 1, 2 ou 3,
   i est égal 2, 3, 4 ou 5, et
   Z représente O, S ou NR₄
   R₄ représentant hydrogène, un radical -(CH₂)ₕ-CH₃ ou - (CF₂)ᵢ-CF₃.

   Parmi les composés de formule (V), on peut notamment citer le méthoxynonafluorobutane vendu sous la dénomination de "MSX 4518^{®}" par la Société 3M (t=1, Z=O, n=0 et m=3) ou l'éthoxynonafluorobutane vendu sous la dénomination de "HFE 7200" par la Société Archimex (t=1, Z=O, n=1 et m=3).
   Les huiles de l'invention ont une température d'ébullition < 220°C de préférence inférieure à 120°C.
   Parmi les cires fluorées à squelette carboné, telles que définies précédemment, susceptibles de former une composition anhydre homogène avec les huiles fluorées, on peut citer notamment les esters fluorés répondant à la formule (VI) suivante : 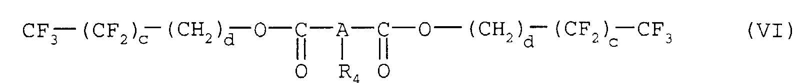 dans laquelle :
   R₄ représente un atome d'hydrogène ou le radical 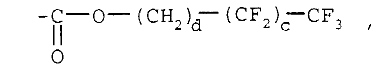
   A représente une chaîne alkylène ou alcénylène, linéaire ou ramifiée, éventuellement hydroxylée, en C₁-C₁₈,
   c est 1 à 17, et
   d est 1 à 18.

   Parmi les composés de formule (VI), on peut notamment citer le dodécane 1,12,dioate de 2F-octyl-éthyle de formule suivante : 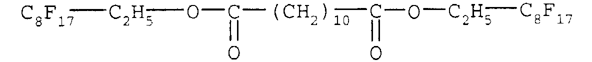 et les tri-citrates de perfluoroalkyle en C₁-C₆, plus particulièrement le tri-citrate de perfluorobutyle vendu sous la dénomination de "ZONYL TBC^{®}" par la Société DUPONT.
   Comme autres cires fluorées utilisables dans l'invention, on peut citer les cires de polytétrafluoroéthylène (PTFE).
   De préférence, on utilise des cires ayant un point de fusion allant de 45°C à 150°C, notamment comme les esters fluorés de formule (VI).
   Dans la composition anhydre selon l'invention, les huiles fluorées sont généralement présentes en une proportion comprise entre 20 et 95 % en poids, mais de préférence entre 60 et 80 % en poids par rapport au poids total de la composition.
   Les cires fluorées à squelette carboné sont généralement présentes en une proportion comprise entre 5 et 80 % en poids, mais de préférence entre 20 et 40 % en poids par rapport au poids total de la composition.
   Selon un mode particulier de réalisation, la composition anhydre telle que définie ci-dessus, peut contenir en outre un ou plusieurs autres corps gras qui peuvent être choisis parmi les huiles, les cires, les gommes et/ou les corps gras dits pâteux.
   **a** - Les huiles de la composition anhydre peuvent être d'origine minérale, animale, végétale ou de synthèse, celles-ci pouvant être volatiles ou non à température ambiante.
      Comme huile d'origine minérale, on peut notamment citer l'huile de paraffine.
      Comme huile d'origine animale, on peut citer notamment le squalène ou perhydrosqualène.
      Comme huile d'origine végétale, on peut citer notamment l'huile d'amande douce, l'huile de calophyllum, l'huile de palme, l'huile d'avocat, l'huile de jojoba, l'huile de sésame, l'huile d'olive, l'huile de ricin et les huiles de germes de céréales telles que par exemple l'huile de germes de blé.
      Comme huile de synthèse, on peut citer notamment :
      (1) les esters de formule :

         R₅-COOR₆

         dans laquelle :
         R₅ représente le reste d'un acide gras supérieur en C₇-C₂₀, et
         R₆ représente un radical hydrocarboné en C₃-C₃₀.
      Parmi ces esters, on peut notamment citer : l'huile de Purcellin, le myristate de butyle, le myristate d'isopropyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate de décyle, le laurate d'hexyle, l'isononanoate d'isononyle, les esters dérivés de l'acide lanolique tels que le lanolate d'isopropyle et le lanolate d'isocétyle.
         Comme autres huiles de synthèse, on peut citer en outre l'isodo-décane, l'isohexadécane, les polyisobutènes et le polyisobutène hydrogéné ainsi que les acétylglycérides, les octanoates et décanoates de polyalcools tels que ceux de glycol et de glycérol, les ricinoléates d'alcools ou de polyalcools tels que le ricinoléate de cétyle, le dicaprylate de propylène glycol et l'adipate de diisopro-pyle;
      (2) les alcools gras tels que l'alcool oléique, l'alcool linoléique, l'alcool linolénique, l'alcool isostéarylique et l'octyldodécanol ;
      (3) les huiles de silicone telles que les polydiorganosiloxanes linéaires éventuellement fonctionnalisés, les polydiorganosiloxanes cycliques et en particulier les cyclotétra- et penta-diméthicones et les organopolysiloxanes tels que les alkyl, alcoxy ou phényl diméthicones et en particulier la phényltriméthi-cone;

   Les huiles peuvent représenter de 0 à 50 % et mieux de 0 à 20 % du poids total de la composition.
**b**- Les cires de la composition anhydre, autres que des cires fluorées, peuvent être d'origine minérale, fossile, animale, végétale, de synthèse ou bien encore être des huiles hydrogénées ou des esters gras concrets à 25°C.
   Parmi les cires minérales, on peut citer notamment les cires microcristallines, la paraffine, la vaseline et la cérésine.
   Parmi les cires fossiles, on peut citer l'ozokérite et la cire de montan.
   Parmi les cires d'origine animale, on peut citer la cire d'abeilles, le spermaceti, la cire de lanoline ainsi que les dérivés issus de la lanoline tels que les alcools de lanoline, la lanoline hydrogénée, la lanoline hydroxylée, la lanoline acétylée, les acides gras de lanoline et l'alcool de lanoline acétylé.
   Parmi les cires d'origine végétale, on peut citer notamment la cire de candellila, la cire de carnauba, la cire du Japon et le beurre de cacao. Parmi les cires de synthèse, on peut citer notamment les homo-polymères d'éthylène et les copolymères d'éthylène et d'un monomère répondant à la formule suivante :

   CH₂=CH-R₇

   dans laquelle :
   R₇ représente un radical en C₁-C₃₀, un radical aryle ou aralkyle ; R₇ étant de préférence un radical méthyle, éthyle, propyle, isopropyle, butyle, dé-cyle, dodécyle ou octadécyle.
   On peut également utiliser des cires obtenues par synthèse Fis-her-Tropsch ainsi que des cires de silicone.
   Parmi les huiles hydrogénées concrètes à 25°C, on peut citer notamment l'huile de ricin hydrogénée, l'huile de palme hydrogénée, le suif hydrogéné et l'huile de coco hydrogénée.
   Parmi les esters gras concrets à 25°C, on peut citer notamment le mono-myristate de propylène glycol et le myristate de myristyle.
   Comme cires utilisables dans les compositions selon l'invention, on peut en outre citer l'alcool cétylique, l'alcool stéarylique, les mono-, di- et triglycérides concrets à 25°C, le monoéthanolamide stéarique, la colophane et ses dérivés tels que les abiétates de glycol et de glycérol, les sucro-glycérides et les oléates, myristates, lanolates, stéarates et dihydroxystéarates de calcium, de magnésium, de zinc et d'aluminium.
   De préférence, on utilise des cires ayant un point de fusion allant de 45°C à 150°C . Elles représentent notamment de 0 à 20 % du poids total de la composition.
**c**- Les corps gras de type pâteux peuvent être d'origine minérale, animale, végétale ou de synthèse.
   Parmi les corps gras pâteux, on peut citer notamment les esters de synthèse tels que le propionate d'arachidyle, le polylaurate de vinyle, les cires de polyéthylène et les organopolysiloxanes tels que les alkyldiméthicones, les alcoxydiméthicones ou les esters de diméthicones.
   La présente invention a également pour objet une composition ou un produit de maquillage ou de soins contenant une composition ou phase anhydre et homogène telle que précédemment définie.
   Selon un premier mode particulier de réalisation, le produit de maquillage ou de soins est anhydre et contient la composition anhydre et homogène en une proportion comprise entre environ 0,5 et 100 % en poids par rapport au poids total du produit.
   Ce produit anhydre peut se présenter sous la forme d'un stick tel que par exemple d'un rouge à lèvres ou d'un anti-cernes, ou sous la forme d'un compact anhydre tel que par exemple d'un fond de teint, d'un fard à paupières, d'un blush ou d'un mascara.
   Lorsque le produit anhydre est sous la forme d'un stick ou d'un compact, celui-ci peut contenir des pigments ainsi que des charges sous forme de fines particules ayant une taille moyenne comprise entre 0,02 et 50 µm.
   Les pigments peuvent être minéraux ou organiques ou encore sous forme de laques métalliques. Parmi ces pigments on peut citer le dioxyde de titane, l'oxyde de zinc, le D&C Red No, 36 et le D&C orange No. 17, les laques de calcium de D&C Red No. 7, 11, 31 et 34, la laque de baryum de D&C Red No. 12, la laque de strontium D&C Red No. 13, les laques d'aluminium de FD&C Yellow No. 5, de FD&C Yellow No. 6, de D&C Red No. 27, de D&C Red No. 21, de FD&C Blue No. l, les oxydes de fer, le violet de manganèse, L'oxyde de chrome et le bleu d'outremer.

Les charges peuvent être d'origine naturelle ou synthétique. Parmi celles-ci, on peut notamment citer :
i) les poudres minérales telles que le talc, le kaolin, le mica, la silice, les silicates, l'alumine, les zéolites, l'hydroxyapatite, la séricite, les micatitanes, le sulfate de baryum, l'oxychlorure de bismuth, le nitrure de bore et les poudres métalliques telles que la poudre d'aluminium ;
ii) les poudres végétales telles que les poudres d'amidon, de maïs, de froment ou de riz ;
iii) les poudres organiques telles que les poudres de Nylon, de polyamide, de polyester, de polytétrafluoroéthylène ou de polyéthylène.

Ces différentes poudres peuvent en outre être enrobées, par exemple, par des sels métalliques d'acides gras, des acides aminés, de la lécithine, du collagène, des composés siliconés, des composés fluorés ou par tout autre enrobage usuel.

En plus des pigments tels que définis ci-dessus, au moins un colorant peut en outre être présent et, parmi ceux-ci, on peut citer les dérivés d'éosine tel que le D&C Red No. 21 et les dérivés de fluoroscéine halogéné tel que le D&C Red No. 27, le D&C orange No. 5 en association avec le D&C Red No. 21 et le D&C orange No. 10.

Dans les produits de maquillage ou de soins, la proportion en pig-ment(s) et/ou colorant(s) est généralement comprise entre environ 0,1 et 25 % et mieux entre 0,1 et 15 % en poids par rapport au poids total de ces produits.

Les charges peuvent être généralement présentes dans les produits de maquillage ou de soins en une proportion maximum d'environ 98 % en poids par rapport au poids total de ces produits.

Les produits anhydres telles que définies ci-dessus, peuvent bien entendu contenir en outre un ou plusieurs additifs ou adjuvants cosmétiques ou dermatologiques conventionnels.

Selon un deuxième mode de réalisation, le produit de maquillage ou de soins est une dispersion, sous forme d'une émulsion stable eau-dans-l'huile (E/H) ou huile-dans-l'eau (H/E), qui est essentiellement constituée (i) d'une composition ou phase anhydre et homogène telle que définie précédemment en une proportion comprise entre environ 0,1 et 50 % en poids par rapport au poids total de la composition, (ii) d'une phase aqueuse en une proportion comprise entre environ 4 et 97 % en poids par rapport au poids total de la composition, et (iii) d'au moins un agent émulsionnant en une proportion comprise entre environ 0,5 et 10 % en poids par rapport au poids total de la dispersion.

Comme agent émulsionnant ou tensioactif pouvant être utilisé dans les compositions sous forme d'une émulsion E/H ou H/E, on peut citer notamment les agents tensioactifs siliconés, et en particulier ceux appartenant à la famille des diméthicones copolyols et des alkyl- ou alcoxydiméthicones copolyols. Parmi ces derniers, on peut citer notamment les composés répondant à la formule générale suivante : 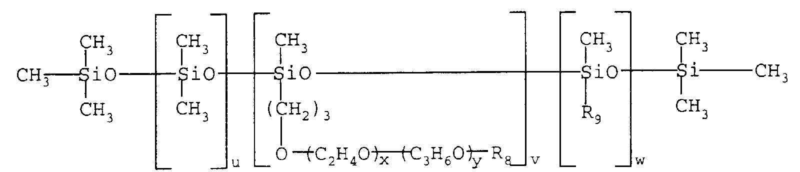 dans laquelle :
R₈ est un atome d'hydrogène, un alkyle en C₁-C₁₆, un alcoxy ou acyle,
R₉ est un radical alkyle ou alcoxy en C₈-C₄₅,
u = 0 à 200,
v = 1 à 40,
w = 0 à 100,
le poids moléculaire du radical -O-(C₂H₄O)ₓ-(C₃H₆O)_{y}-R₈ étant de 250 à 2000, x et y étant choisis de telle sorte que le rapport en poids des groupes oxyéthy-lène/oxypropylène soit compris entre 100:0 et 20:80.

Parmi les produits du commerce pouvant contenir tout ou partie des al-kyldiméthicones copolyols, on peut citer notamment ceux vendus sous les dénominations de "ABIL WE09^{®}", "ABIL EM90^{®}" ou "ABIL WS08^{®}" par la Société GOLDSCHMIDT, de "Q2 5200^{®}" ou "Q2 3225C^{®}" par la Société DOW CORNING et de "218 |138®" par la Société GENERAL ELECTRIC.

Les agents tensioactifs peuvent être également choisis parmi des agents tensioactifs anioniques ou non ioniques. On peut à ce sujet se reporter au document "Encyclopedia of Chemical Technology, KlRK-OTHMER", volume 22, pages 333-432, 3^{ème} édition, 1979 WILEY, pour la définition des propriétés et des fonctions (émulsionnant) des tensioactifs, en particulier pages 347-377 de cette référence, pour les tensioactifs anioniques et non ioniques.

Les tensioactifs de ces deux groupes utilisés de préférence dans les compositions selon l'invention sont :
- parmi les tensioactifs non ioniques : les acides gras, les alcools gras, les alcools gras polyéthoxylés ou polyglycérolés, tels que les alcools stéarylique ou cétylstéarylique polyéthoxylés, les esters d'acides gras et de saccharose, les esters alkyl glucose, en particulier les esters gras d'alkyl(C₁ -C₆)glucose polyoxy-éthylénés, et
- parmi les tensioactifs anioniques : les stéarates d'amines.

Ces dispersions se présentent de préférence sous forme de crèmes et peuvent être utilisées comme produits de maquillage ou de soins.

Les produits ou compositions tels que décrits ci-dessus, qu'ils soient du type anhydre ou sous forme d'une dispersion, présentent d'excellentes propriétés cosmétiques telles que notamment une excellente texture, ainsi qu'une très bonne tenue sur la peau vis-à-vis des frottements, de l'eau ou du sébum.

Ces produits ou compositions tels qu'ils viennent d'être décrits ci-dessus, peuvent en outre contenir un ou plusieurs adjuvants cosmétiques conventionnels tels que des vitamines, des hormones, des agents antioxydants, des conservateurs, des parfums, des épaississants, des agents hydratants, des agents humectants, des polymères anioniques, non-ioniques ou amphotères, des actifs cosmétiques ou dermatologiques.

L'invention est illustrée par les exemple suivants, dont les quantités sont exprimées en poids.

### EXEMPLES de COMPOSITION COSMETIQUE

*Exemple 1*: **Rouge à lèvres**
   On prépare selon l'invention, un rouge à lèvres en procédant au mélange des ingrédients suivants :
   - Polyperfluoroisopropyléther vendu sous la dénomination de "FLUORTRESS LM 36^{®}" par la Société
   - DUPONT 5 g
   - Dodécane 1,12 dioate de 2F-octyl-éthyle 40 g
   - Perfluorodécaline 35 g
   - Méthoxynonafluorobutane vendu sous la dénomination de "MS X 4518^{®}" par la Société 3M 10 g
   - Pigments 10
*Exemple 2*: **Fond de teint en stick**
   On prépare selon l'invention, un fond de teint en procédant au mélange des ingrédients suivants :
   - Dodécane 1,12 dioate de 2F-octyl-éthyle 30 g
   - N-(-2-F-octyl-éthyloxycarbonyl)-3-amino-propyl bis(triméthylsiloxy)méthylsilane 60 g
   - Pigments 10 g
*Exemple 3*: Crème de soin
   On prépare une émulsion eau-dans-huile avec les ingrédients suivants selon les méthodes classiques de fabrication des émulsions :
   - FLUORTRESS LM 36 (1) g
   - Zonyl TBC (2) 8 g
   - Tri-fluorométhyl C₁₋₄ alkyl diméthicone (Shin
   - Etsu) 12 g
   - Abil WE 09 (3) 5 g
   - Sel 0,7 g
   - Eau qps100 g
      (1) polyperfluoroisopropyl éther (Dupont de Nemours)
      (2) tri-perfluoroalkyl éthyl citrate (Dupont de Nemours)
      (3) polyglycéryl-4 isostéarate/cétyl diméthicone copolyol/hexyl laurate
      (Goldschmidt).

## Revendications

1. Composition anhydre et homogène, caractérisée par le fait qu'elle comprend en association au moins une huile fluorée et au moins une cire fluorée à squelette carboné.

2. Composition selon la revendication 1, caractérisée par le fait que l'huile fluorée est un composé perfluoré choisi parmi:
a) les perfluoropolyéthers répondant aux formules (l) et (ll) suivantes : 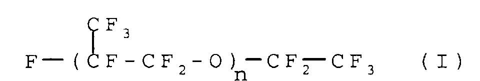 dans laquelle :
n est 7 à 30; et 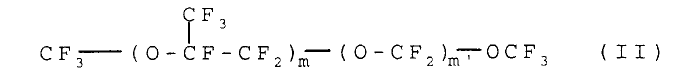
le rapport m/m' étant de 20 à 40, et
le poids moléculaire étant de 500 à 20000 ;
b) les composés perfluorocycloalkyles de formule (III) suivante :
(CF₂)ₑ (CF-(CF₂)ₚ-F)_{g} (III)
dans laquelle :
e est égal à 4 ou 5,
g est égal à 1 ou 2, et
p est égal à 1, 2 ou 3 ;
sous réserve que lorsque g=2, les groupements ne sont pas nécessairement en alpha l'un par rapport à l'autre ; et
c) le perfluorophénanthrène.

3. Composition selon la revendication 1, caractérisée par le fait que l'huile fluorée est un composé fluoro-siliconé répondant à la formule (IV) suivante: 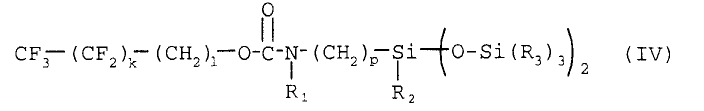 dans laquelle :
k est 1 à 17,
l est 1 à 18,
p est 1 à 6, et
Rᵢ représente un atome d'hydrogène ou un radical alkyle en C₁-C₆,
R₂ représente un radical alkyle en C₁-C₆ ou le radical -OSi(R₃)₃, et
R₃ représente un radical alkyle en C₁-C₄.

4. Composition selon la revendication 3, caractérisée par le fait que le composé fluoro-siliconé est choisi dans le groupe constitué par :
- le N-(-2-F-octyl-éthyloxycarbonyl)-3-aminopropyl bis(triméthylsiloxy)méthylsilane,
- le N-(-2-F-hexyl-éthyloxycarbonyl)-3-aminopropyl bis(triméthylsiloxy)méthylsilane,
- le N-(-2-F-butyl-éthyloxycarbonyl)-3-aminopropyl bis(triméthylsiloxy)méthylsilane,
- le N-(-2-F-octyl-éthyloxycarbonyl)-3-aminopropyl tris(triméthylsiloxy)silane,
- le N-(-2-F-hexyl-éthyloxycarbonyl)-3-aminopropyl tris(triméthylsiloxy)silane, et
- le N-(-2-F-butyl-éthyloxycarbonyl)-3-aminopropyl tris(triméthylsiloxy)silane.

5. Composition selon la revendication 1, caractérisée par le fait que l'huile fluorée est un composé fluoroalkyle ou hétérofluoroalkyle répondant à la formule (V) suivante:
CH₃-(CH₂)ₕ-[Z]ₜ-(CF₂)ᵢ-CF₃ (V)
dans laquelle :
t est égal à 0 ou 1,
h est égal à 0, 1, 2 ou 3,
i est égal 2, 3, 4 ou 5, et
Z représente O, S ou NR₄
R₄ représentant hydrogène, un radical -(CH₂)ₕ-CH₃ ou -(CF₂)ᵢ-CF₃.

6. Composition selon les revendications 1 à 5, caractérisée par le fait que la cire fluorée à squelette carboné est choisie parmi les esters fluorés répondant à la formule (VI) suivante: 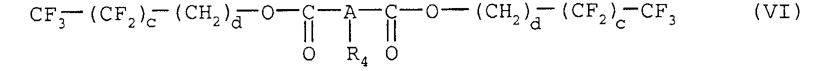 dans laquelle :
R₄ représente un atome d'hydrogène ou le radical 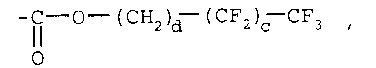
A représente une chaîne alkylène ou alcénylène, linéaire ou ramifiée, éventuellement hydroxylée, en C₁-C₁₈,
c est 1 à 17, et
d est 1 à 18.

7. Composition selon l'une quelconque des revendications 1 à 6, caractérisée par le fait que la cire fluorée à squelette carboné est choisie dans le groupe constitué par le dodécane 1,12 dioate de 2F-octyl-éthyle et le tri-citrate de perfluorobutyle.

8. Composition selon l'une quelconque des revendications précédentes, caractérisée par le fait que l'huile fluorée est présente en une proportion comprise entre 20 et 95 % en poids par rapport au poids total de la composition.

9. Composition selon l'une quelconque des revendications précédentes, caractérisée par le fait que la cire fluorée à squelette carboné est présente en une proportion comprise entre 5 et 80 % en poids par rapport au poids total de la composition.

10. Produit de maquillage ou de soins contenant au moins une composition anhydre et homogène, caractérisé par le fait que ladite composition est telle que définie selon l'une quelconque des revendications 1 à 9.

11. Produit selon la revendication 10, caractérisé par le fait que ladite composition est présente en une proportion comprise entre 0,5 et 100 % en poids par rapport au poids total du produit.

12. Produit selon les revendications 10 et 11, caractérisé par le fait qu'il est anhydre et se présente sous forme d'un stick ou d'un compact.

13. Produit selon les revendications 10 et 11, caractérisé par le fait qu'il se présente sous forme d'une dispersion ou émulsion du type eau-dans-huile (E/H) ou huile-dans-eau (H/E) et contient ladite composition en une proportion comprise entre 0,1 et 50 % en poids, une phase aqueuse présente en une proportion comprise entre 4 et 97 %, et un agent émulsionnant.

14. Produit selon l'une quelconque des revendications 10 à 13, caractérisé par le fait qu'il contient en outre au moins un pigment et/ou un colorant en une proportion comprise entre 0,1 et 15 % en poids par rapport au poids total du produit.

15. Produit selon l'une quelconque des revendications 10 à 14, caractérisé par le fait qu'il contient en outre une charge présente en une proportion maximum de 98 % en poids par rapport au poids total du produit.

16. Produit selon l'une quelconque des revendications 10 à 15, caractérisé par le fait qu'il contient en outre des actifs cosmétiques ou dermatologiques.
